Europäisches Patentamt

European Patent Office

Office européen des brevets

(11) Numéro de publication: **0 184 530**

**A1**

(12) # DEMANDE DE BREVET EUROPEEN

(21) Numéro de dépôt: **85420215.7**

(22) Date de dépôt: **02.12.85**

(51) Int. Cl.⁴: **A 61 N 5/06**

(30) Priorité: **07.12.84 FR 8418957**

(43) Date de publication de la demande:
**11.06.86 Bulletin 86/24**

(84) Etats contractants désignés:
**BE CH DE IT LI NL**

(71) Demandeur: **Société Coopérative Ouvrière de Production à Responsabilité Limitée: ERIEL - Société d'Engineering et de Réalisations Electroniques et Industrielles Vareilles F-48230 Chanac(FR)**

(72) Inventeur: **Hrebicek, Erick Le Villard F-48230 Chanac(FR)**

(72) Inventeur: **Penel, Christian Le Villard F-48230 Chanac(FR)**

(74) Mandataire: **Ropital-Bonvarlet, Claude et al, Cabinet BEAU DE LOMENIE 99, Grande rue de la Guillotière F-69007 Lyon(FR)**

(54) Appareil de traitement thérapeutique par application d'un faisceau laser.

(57) - Appareil de traitement thérapeutique par application d'un faisceau laser sur une partie du corps humain.

- L'appareil comprend un générateur laser à gaz (10) émettant un faisceau laser principal (100) de lumière cohérente visible, un dispositif optique (20) pour subdiviser le faisceau laser principal (100) en une pluralité de faisceaux parallèles (101 à 106) situés dans un même plan, une pluralité de sources lasers à semi-conducteurs (31 à 36) émettant un pluralité de faisceaux lasers infrarouge parallèles situés dans un plan parallèle au plan des faisceaux lasers visibles (101 à 106) et des moyens pour déplacer l'ensemble de la tête d'émission dans une direction perpendiculaire aux plans des faisceaux lasers parallèles émis.

Fig-2b

"Appareil de traitement thérapeutique par application d'un faisceau laser" :

La présente invention a pour objet un appareil de traitement thérapeutique, par application d'un faisceau de lumière cohérente sur une zone déterminée du corps humain, comprenant un générateur laser à gaz émettant un faisceau principal de lumière cohérente dans le domaine visible et des moyens de déviation du faisceau principal émis par le générateur laser à gaz.

On connaît déjà l'utilisation d'appareils laser dans le domaine médical à des fins de traitements thérapeutiques autres que des interventions chirurgicales. Des faisceaux laser peuvent ainsi être utilisés, notamment pour le traitement de phénomènes inflammatoires, par exemple tendinites.

Selon un premier mode de traitement, on utilise un simple générateur laser à gaz, du type He - Ne, pour créer un faisceau de lumière cohérente visible qui est dirigé sur la partie du corps humain à traiter. Le faisceau de lumière cohérente visible est, cependant, focalisé sur une zone ponctuelle très limitée du corps humain et il est nécessaire qu'un manipulateur agisse sur des moyens de déviation du faisceau laser pour déplacer la zone ponctuelle d'impact du faisceau laser, de manière à agir sur l'ensemble de la surface à traiter. On sait, en effet, que l'action thérapeutique créée par l'impact d'un faisceau laser visible sur le corps ne s'étend que sur une zone relativement peu étendue, de l'ordre de 2 cm de diamètre autour du point d'impact.

Pour éviter de procéder à un décalage manuel du faisceau laser, on a déjà proposé de provoquer un balayage automatique de la zone à traiter par le faisceau laser. Ce balayage est réalisé à l'aide de miroirs oscillants opérant le renvoi du faisceau laser émis par le générateur laser. Ce système permet bien de couvrir une zone étendue du corps humain. Toutefois, l'effet thérapeutique est fortement atténué lorsque l'impact du faisceau laser sur la zone à traiter est trop bref. Pour obtenir une action efficace, on est ainsi conduit à augmenter la puissance du générateur laser, ce qui accroît le coût du procédé et peut présenter des dangers pour le patient. D'une manière générale, les lasers à balayage connus à ce jour entraînent des pertes d'énergie importantes.

On a encore proposé d'adjoindre, à un faisceau laser visible créé par un générateur He - Ne, un faisceau laser infra-rouge obtenu à partir d'une source laser à semi-conducteur, telle qu'une source As Ga. Un inconvénient majeur de cette technique réside dans le fait qu'un faisceau émis, à partir d'une source laser As Ga, n'est pas visible, ce qui provoque une impossibilité d'apprécier exactement l'étendue de la zone traitée.

La présente invention a, précisément, pour objet de remédier aux inconvénients précités et de permettre la couverture automatique précise et efficace de l'ensemble d'une zone à soumettre à l'impact de faisceaux laser à des fins de traitement thérapeutique.

L'invention a encore pour objet de permettre de réaliser le traitement d'une partie du corps humain avec un bon rendement et un appareillage peu complexe.

Ces buts sont atteints grâce à un appareil de traitement thérapeutique du type défini en tête de la description qui, conformément à l'invention, comprend des moyens pour subdiviser le faisceau laser principal en une pluralité de faisceaux parallèles de lumière cohérente visible situés dans un même plan et des moyens pour déplacer simultanément le générateur laser à gaz et les moyens de subdivision du faisceau laser principal selon une direction perpendiculaire au plan contenant ladite pluralité de faisceaux parallèles.

Grâce à la subdivision du faisceau laser principal en une pluralité de faisceaux parallèles, il est créé à chaque instant, simultanément, une série de points d'impact de faisceaux laser sur la zone à traiter, ce qui permet d'exercer en chaque point une action prolongée plus efficace que celle obtenue lors d'un balayage, tout en couvrant à chaque instant une partie relativement étendue de la zone à traiter. Le déplacement linéaire par pas, selon une direction donnée, de l'ensemble de la tête d'émission de rayonnement, permet de couvrir facilement l'ensemble de la zone à traiter, sans qu'il soit nécessaire de disposer de mécanismes complexes de déviation ou de guidage.

Avantageusement, les moyens de subdivision du faisceau laser principal comprennent un ensemble de miroirs semi-réfléchissants subdivisant chacun un faisceau incident en un faisceau réfléchi et un faisceau transmis et au moins un premier miroir de renvoi.

De préférence, les moyens de subdivision du faisceau laser principal sont agencés de manière à créer une pluralité de faisceaux parallèles coplanaires équidistants les uns des autres.

L'efficacité de l'appareil selon l'invention peut encore être accrue grâce à la mise en oeuvre supplémentaire d'une pluralité de générateurs lasers à semi-conducteur émettant une lumière cohérente dans le domaine de l'infra-rouge et alignés au voisinage des moyens de subdivision du faisceau laser principal du générateur laser à gaz, de manière à produire une pluralité de faisceaux parallèles de lumière cohérente infra-rouge situés dans un plan parallèle au plan contenant la pluralité de faisceaux parallèles de lumière visible, chaque faisceau parallèle de lumière cohérente infra-rouge étant situé en regard d'un faisceau correspondant de lumière cohérente visible.

Dans ce cas, chaque générateur laser à semi-conducteur comprend une diode laser et des moyens optiques de collimation destinés à créer un faisceau de lumière cohérente infra-rouge présentant une section de surface et de contour prédéterminés.

Selon ce mode de réalisation, les faisceaux de lumière cohérente visible issus des moyens de subdivision du faisceau principal produit par le générateur laser à gaz contribuent, grâce à leur positionnement particulier, non seulement à faciliter le déroulement du traitement thérapeutique, mais également à repérer et donc à contrôler l'action des faisceaux de lumière cohérente infra-rouge invisible, du fait des positions relatives préétablies de ces derniers par rapport aux faisceaux de lumière cohérente visible.

De préférence, la distance entre deux faisceaux parallèles de lumière visible est du même ordre de grandeur que la distance entre un faisceau de lumière visible et un faisceau de lumière infra-rouge situés en regard l'un de l'autre et peut être de l'ordre de 2 cm.

D'autres caractéristiques et avantages de l'invention ressortiront de la description suivante de modes particuliers de réalisation de l'invention, en référence aux dessins annexés sur lesquels :

- les fig. 1a, 1b et 1c représentent, respectivement, des vues de côté, de dessous et de face de l'ensemble d'émission de rayonnement laser d'un appareil conforme à l'invention, selon un premier mode de réalisation utilisant un tube laser à gaz,

- les fig. 2a et 2b représentent, respectivement, des vues de côté et de dessous de l'ensemble d'émission de rayonnement laser d'un appareil conforme à l'invention, selon un second mode de réalisation utilisant à la fois un tube laser à gaz et une source laser à semi-conducteur,

- les fig. 3 et 4 représentent deux exemples d'images pouvant être produites à un instant donné sur une zone à traiter avec une tête d'émission conforme au mode de réalisation des fig. 2a et 2b, et,

- les fig. 5 et 6 représentent, respectivement, en vue de côté et en vue arrière, un exemple de moyens d'entraînement pas à pas et de guidage de têtes d'émission des fig. 1a à 1c et 2a, 2b.

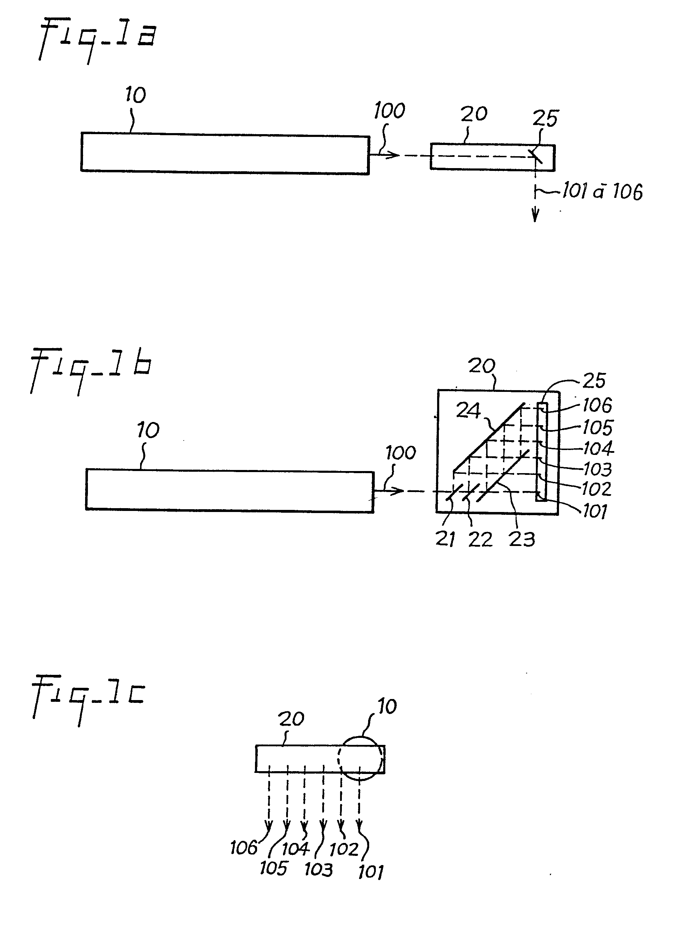

On voit, sur les fig. 1a à 1c, un premier exemple de tête d'émission de rayonnement laser conforme à l'invention, utilisable à des fins thérapeutiques. Cette tête d'émission comprend une source laser à gaz 10 constituée par un tube générateur du type He - Ne produisant un faisceau principal 100 de lumière cohérente, dont la longueur d'onde se situe dans le domaine visible. Le faisceau principal 100 est appliqué à un dispositif optique 20 comprenant des moyens pour subdiviser le faisceau principal 100 en un nombre $n$ de faisceaux secondaires 101 à 106. Sur l'exemple représenté, le nombre $n$ est égal à six. Le nombre $n$ est, avantageusement, compris entre 4 et 8, mais pourrait être un nombre entier supérieur.

Le dispositif optique 20 comprend, essentiellement, un ensemble de miroirs semi-réfléchissants 21, 22, 23 délivrant chacun, à partir d'un rayon incident, d'une part, un premier rayon produit par réflexion et, d'autre part, un second rayon produit par transmission. Des miroirs de renvoi 24, 25 complètent l'ensemble de miroirs semi-réfléchissants 21, 22, 23 pour permettre la production d'un ensemble de rayons parallèles coplanaires en sortie du dispositif optique 20.

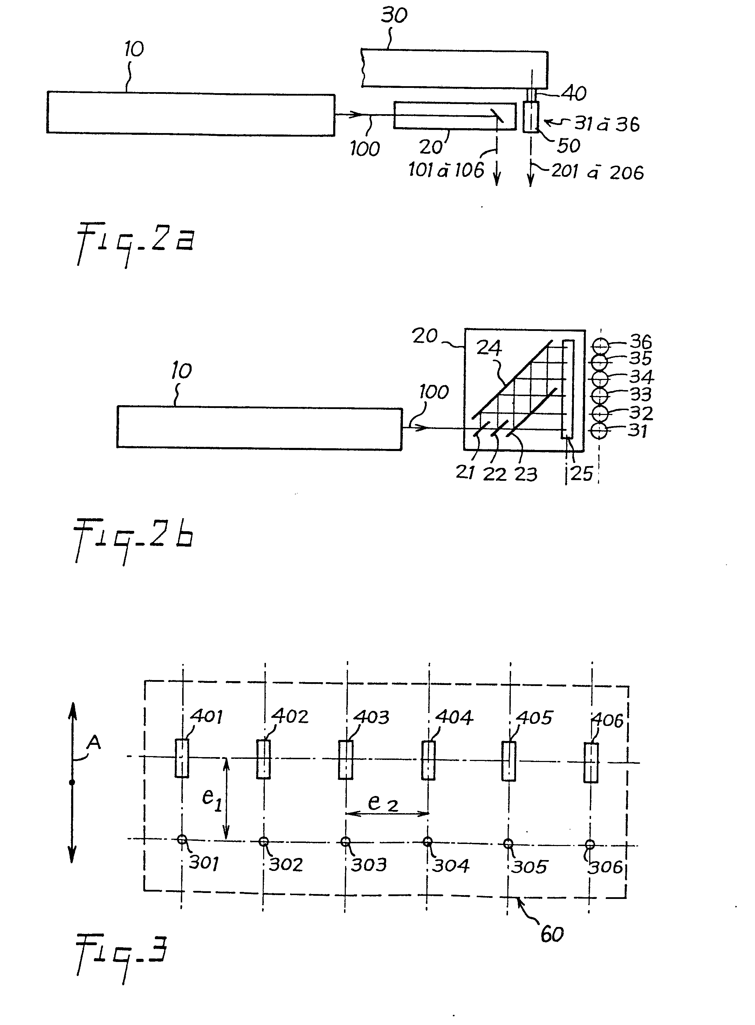

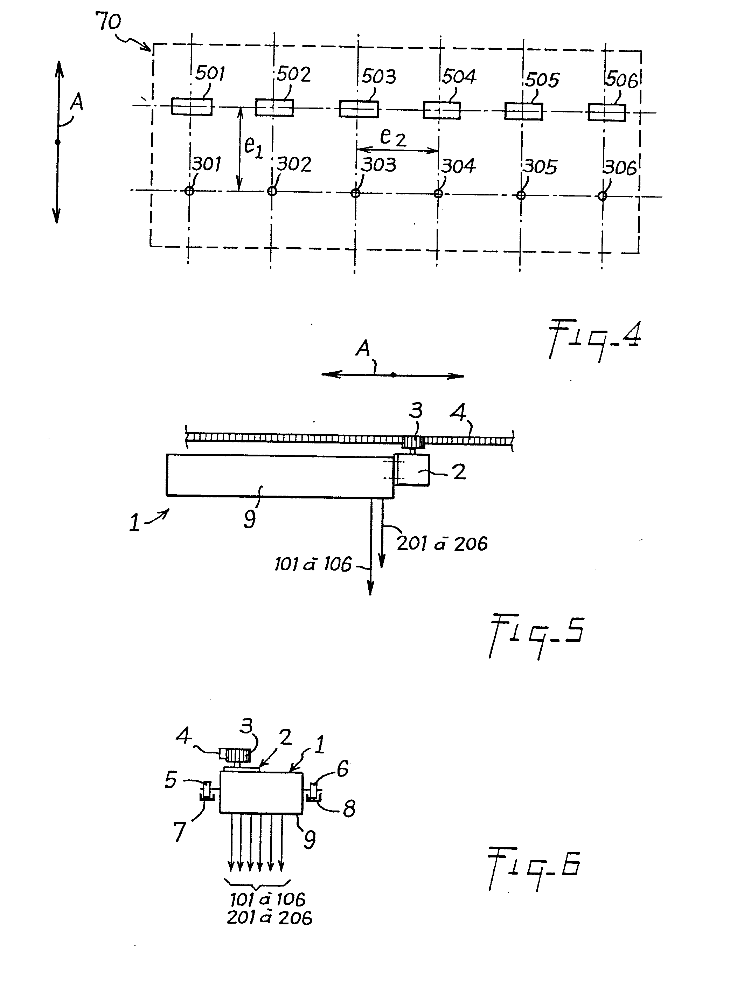

Sur la fig. 1b, on voit un exemple de dispositif optique 20 adapté pour produire six rayons 101 à 106, à partir d'un rayon laser principal incident 100. Le dispositif 20 comprend un premier miroir semi-réfléchissant 21 incliné à 45° par rapport à la direction du faisceau principal 100, un second miroir semi-réfléchissant 22 parallèle au premier miroir semi-réfléchissant 21 et disposé de manière à diviser en deux le rayon transmis par le premier miroir semi-réfléchissant 21 et un troisième miroir semi-réfléchissant 23 disposé parallèlement au second miroir semi-réfléchissant 22, de manière à diviser encore en deux le rayon transmis par ce dernier. Un miroir plan 24 est disposé parallèlement aux miroirs semi-réfléchissants 21, 22, 23, de manière à renvoyer les rayons réfléchis par ces miroirs dans une direction parallèle à celle du rayon 101 transmis par le troisième miroir semi-réfléchissant 23. Ce dernier miroir 23 est prolongé parallèlement au miroir 24, de manière à provoquer une nouvelle subdivision des rayons réfléchis par les miroirs semi-réfléchissants 21 et 22, puis par la partie du miroir 24 située en regard des miroirs semi-réfléchissants 21 et 22. Le miroir semi-réfléchissant prolongé 23 assure ainsi une nouvelle subdivision de rayons réfléchis pour former, par transmission, des rayons 102, 103 parallèles au rayon 101 transmis à travers la succession de miroirs semi-réfléchissants 21, 22, 23 et, par réflexion après renvoi sur le miroir plan 24 situé en regard du miroir semi-réfléchissant 23, des rayons 105, 106, également parallèles au rayon 101. Le rayon de sortie 104 est lui-même formé par le premier rayon réfléchi par le miroir semi-réfléchissant 23 et renvoyé par le miroir de renvoi 24. Il est ainsi produit une série de rayons ou faisceaux parallèles 101 à 106 issus du même faisceau laser principal 100. Un miroir plan de sortie 25 présente un plan incliné de 45° par rapport au faisceau principal 100 et aux plans des miroirs 21, 22, 23 et 24, de manière à renvoyer les n faisceaux laser parallèles subdivisés 101 à 106 dans un plan perpendiculaire au faisceau principal 100. Dans le cas des fig. la à lc, on a représenté des faisceaux subdivisés 101 à 106 orientés verticalement vers le bas, de manière à former, sur une zone à traiter disposée sensiblement horizontalement sous la tête d'émission laser, une série de points d'impact visibles alignés et équidistants 301 à 306 (fig. 3 et 4).

Les fig. 2a et 2b montrent un second mode de réalisation d'un appareil selon l'invention, dans lequel la source laser à gaz 10 et le dispositif optique 20 sont conformes à la description donnée en référence aux fig. la à lc pour former une pluralité de faisceaux laser parallèles 101 à 106 coplanaires et équidistants, à partir d'un faisceau principal 100 de lumière visible. Toutefois, un ensemble 30 est associé au dispositif optique 20 et à la source laser à gaz 10 pour produire une série de faisceaux lasers supplémentaires (201 à 206) dans le domaine de l'infra-rouge. Cette source laser supplémentaire comprend plusieurs sources élémentaires alignées 31 à 36, montées sur un support et comprenant chacune une diode laser 40, par exemple du type As Ga, associée à un dispositif optique de collimatation 50 destiné à former sur une distance de, par exemple, 2 m, un faisceau de rayons quasi parallèles présentant une section de contour et de surface prédéterminés.

Les sources lasers élémentaires 31 à 36 à semi-conducteur sont alignées, de manière à produire des faisceaux 201 à 206 parallèles et équidistants situés dans un plan parallèle au plan contenant les faisceaux laser visibles 101 à 106. De façon plus particulière, le nombre n de faisceaux laser infra-rouge 201 à 206 correspond exactement au nombre n de faisceaux laser visibles 101 à 106 et chaque faisceau laser infra-rouge 201 à 206, respectivement, est situé en regard d'un faisceau laser visible 101 à 106, respectivement. Les faisceaux laser visibles 101 à 106 ont ainsi, non seulement une fonction de traitement du corps humain, mais présentent un rôle de repérage des points d'impact des faisceaux laser 201 à 206 invisibles de lumière cohérente infra-rouge.

Les fig. 3 et 4 montrent deux exemples d'images que l'on peut obtenir sur une zone à traiter avec une série de faisceaux lasers visibles 101 à 106 et une série de faisceaux lasers infra-rouges 201 à 206.

On notera que l'image obtenue par collimatation du faisceau de sortie d'une diode laser 40, qui peut être par exemple rectangulaire, reste sensiblement identique à partir de la sortie du dispositif optique de collimatation, sur une distance d'environ 2 m. Ceci permet d'éviter un réglage vertical de la tête d'émission par rapport à un plan de traitement, ce qui autorise l'utilisation d'une structure simplifiée de support de tête d'émission.

On voit, sur les fig. 3 et 4, que les images rectangulaires invisibles 401 à 406, 501 à 506, des faisceaux laser infra-rouge 201 à 206 collimatés sont disposées selon une ligne parallèle à la ligne définie par les images ponctuelles 301 à 306 des faisceaux laser de lumière cohérente visible. Dans la mesure où chaque image rectangulaire 401 à 406, 501 à 506 est située en regard d'une image ponctuelle visible 301 à 306, à une distance prédéterminée $e_1$, les images ponctuelles visibles 301 à 306 jouent ainsi un rôle de matérialisation latérale de la ligne constituée par les taches invisibles des faisceaux laser infra-rouge collimatés. Il est ainsi possible, grâce à la grille constituée par les taches ponctuelles visibles 301 à 306 et à la connaissance des écartements prédéterminés entre taches visibles et invisibles, de surveiller en permanence la zone du corps humain qui est en cours de traitement.

Les images rectangulaires des faisceaux lasers infra-rouges 201 à 206 peuvent avoir leur grand côté perpendiculaire au plan contenant les faisceaux parallèles de lumière cohérente visible 101 à 106 créant les taches ponctuelles 301 à 306 (références 401 à 406 de la fig. 3) ou avoir leur grand côté parallèle à ce plan contenant les faisceaux parallèles de lumière cohérente visible 101 à 106 (références 501 à 506 de la fig. 4).

Compte tenu du fait que la zone d'influence autour d'une tache ponctuelle 301 à 306, créée par un faisceau laser visible, présente un rayon de l'ordre de 1 cm, l'écartement $e_2$ entre deux

images ponctuelles adjacentes 301 à 306 est avantageusement de l'ordre de 2 cm. L'écartement entre les centres de deux images adjacentes 401 à 406, 501 à 506, créées par les faisceaux lasers infra-rouges, correspond lui-même à l'écartement $e_2$ entre deux images ponctuelles adjacentes 301 à 306. L'écartement $e_1$ entre la ligne des images 401 à 406 ou 501 à 506 des faisceaux lasers infra-rouges collimatés 201 à 206 et la ligne des images 301 à 306 des rayons lasers visibles 101 à 106, est avantageusement du même ordre de grandeur que l'écartement $e_2$.

Afin de traiter une zone étendue du corps humain, la tête d'émission représentée sur les fig. 1a à 1c ou 2a, 2b, est déplacée en translation pas à pas horizontalement, de manière à déplacer l'ensemble des images des fig. 3 et 4 dans le sens des flèches A. L'avance pas à pas, par exemple par pas de 1 ou 2 cm, permet de traiter une zone étendue avec une plus grande efficacité qu'un dispositif à balayage continu, tout en ne nécessitant que des moyens d'entraînement et de guidage très simples.

On a représenté, sur les fig. 5 et 6, un exemple de moyens d'entraînement et de guidage de l'ensemble (1) de la tête d'émission constituée du générateur laser à gaz 10, du dispositif optique 20 de subdivision du faisceau laser principal 100 et, le cas échéant, (fig. 2a et 2b) de l'ensemble 30 des sources lasers à semi-conducteurs 31 à 36. Les systèmes d'alimentation et de commandes électriques et électroniques des sous-ensembles de la tête d'émission 1, non représentés sur le dessin, sont également inclus dans le boîtier 9 de l'ensemble 1. Le boîtier 9 est équipé de galets latéraux 5, 6 roulant dans des profilés fixes 7, 8 formant coulisses disposés de part et d'autre de la tête d'émission 1 pour servir de moyens de guidage en translation. Un moteur 2, solidaire du boîtier 9, entraîne une roue dentée 3 qui est en prise avec une crémaillère fixe 4 pour produire un déplacement en translation de la tête d'émission 1 dans le sens de la flèche A qui est perpendiculaire aux plans, de préférence verticaux, qui contiennent les rayons lasers visibles 101 à 106 et les rayons infra-rouges 201 à 206. Le moteur 2 peut être un moteur pas à pas ou un moteur à courant continu piloté avec un système

**0184530**

de détection opto-électronique.

Naturellement, d'autre moyens de guidage et d'entraînement peuvent être envisagés. Ainsi, l'entraînement peut être réalisé
par un système écrou et vis sans fin ou un système à poulie et courroie. Le moteur d'entraînement 2 peut, également, être monté sur le
support fixe portant les profilés 7, 8 plutôt que sur le boîtier 9,
la crémaillère 4 étant alors solidaire du boîtier 9.

Le temps $t$, pendant lequel un ensemble image 60 (fig. 3)
ou 70 (fig. 4) est projeté au même endroit de la zone à traiter à
chaque déplacement d'un pas, est réglé en fonction du traitement à
effectuer.

L'invention n'est pas limitée aux exemples décrits et
représentés, car diverses modifications peuvent y être apportées sans
sortir de son cadre.

0184530

REVENDICATIONS :

1 - Appareil de traitement thérapeutique par application d'un faisceau de lumière cohérente sur une zone déterminée du corps humain, comprenant un générateur laser à gaz (10) émettant un faisceau principal de lumière cohérente (100) dans le domaine visible et des moyens de déviation du faisceau principal (100) émis par le générateur laser à gaz (10),

caractérisé en ce qu'il comprend des moyens (20) pour subdiviser le faisceau laser principal (100) en une pluralité de faisceaux parallèles (101 à 106) de lumière cohérente visible situés dans un même plan et des moyens (2 à 8) pour déplacer simultanément le générateur laser à gaz (10) et les moyens (20) de subdivision du faisceau laser principal (100) selon une direction perpendiculaire au plan contenant ladite pluralité de faisceaux parallèles (101 à 106).

2 - Appareil selon la revendication 1, caractérisé en ce que les moyens (20) de subdivision du faisceau laser principal (100) comprennent un ensemble de miroirs semi-réfléchissants (21, 22, 23) subdivisant chacun un faisceau incident en un faisceau réfléchi et un faisceau transmis et au moins un premier miroir de renvoi (24).

3 - Appareil selon la revendication 2, caractérisé en ce que l'ensemble de miroirs semi-réfléchissants (21, 22, 23) comprend un premier miroir semi-réfléchissant (21) incliné à 45° par rapport à la direction du faisceau principal incident (100) et, au moins, des seconds miroirs semi-réfléchissants (22, 23) disposés respectivement sur les trajets des faisceaux transmis et réfléchis par le premier miroir semi-réfléchissant (21).

4 - Appareil selon la revendication 2 ou 3, caractérisé en ce que les miroirs semi-réfléchissants (21, 22, 23) sont parallèles entre eux et inclinés de 45° par rapport à la direction du faisceau principal incident (100) et en ce que le premier miroir de renvoi (24) est parallèle aux miroirs semi-réfléchissants (21, 22, 23).

5 - Appareil selon l'une quelconque des revendications 2 à 4, caractérisé en ce qu'il comprend un second miroir de renvoi (25), situé dans un plan incliné à 45° par rapport au plan du premier miroir semi-réfléchissant (21).

6 - Appareil selon l'une quelconque des revendications 1 à 5, caractérisé en ce que les moyens (20) de subdivision du faisceau laser principal (100) sont agencés de manière à créer une pluralité de faisceaux parallèles (101 à 106) coplanaires équidistants les uns des autres.

7 - Appareil selon l'une quelconque des revendications 1 à 6, caractérisé en ce qu'il comprend, en outre, une pluralité de générateurs lasers à semi-conducteur (31 à 36) émettant une lumière cohérente dans le domaine de l'infra-rouge et alignés au voisinage des moyens (20) de subdivision du faisceau laser principal (100) du générateur laser à gaz, de manière à produire une pluralité de faisceaux parallèles (201 à 206) de lumière cohérente infrarouge situés dans un plan parallèle au plan contenant la pluralité de faisceaux parallèles (101 à 106) de lumière visible, chaque faisceau parallèle (201 à 206) de lumière cohérente infra-rouge étant situé en regard d'un faisceau correspondant (101 à 106) de lumière cohérente visible.

8 - Appareil selon la revendication 7, caractérisé en ce que chaque générateur laser à semi-conducteur (31 à 36) comprend une diode laser (40) et des moyens optiques de collimation (50) destinés à créer un faisceau de lumière cohérente infra-rouge présentant une section de surface et de contour prédéterminés (401 à 406, 501 à 506).

9 - Appareil selon la revendication 8, caractérisé en ce que les moyens optiques de collimation (50) des générateurs laser à semi-conducteur (31 à 36) sont disposés de manière à créer des faisceaux de lumière cohérente infra-rouge présentant une section rectangulaire (401 à 406) dont le grand côté est perpendiculaire au plan contenant les faisceaux parallèles de lumière cohérente visible (101 à 106).

10 - Appareil selon la revendication 8, caractérisé en ce que les moyens optiques de collimation (50) des générateurs laser à semi-conducteur (31 à 36) sont disposés de manière à créer des faisceaux de lumière cohérente infra-rouge présentant une section rectangulaire (501 à 506) dont le grand côté est parallèle au plan contenant les faisceaux parallèles de lumière cohérente visible (101 à 106).

11 - Appareil selon l'une quelconque des revendications 7 à 10, caractérisé en ce que la distance ($e_2$) entre deux faisceaux parallèles de lumière visible est du même ordre de grandeur que la distance ($e_1$) entre deux faisceaux de lumière visible et de lumière infra-rouge situés en regard l'un de l'autre.

12 - Appareil selon l'une quelconque des revendications 1 à 11, caractérisé en ce que les moyens (2 à 8) pour déplacer, simultanément, l'ensemble (1) constitué par le générateur laser à gaz (10), les moyens (20) de subdivision du faisceau laser principal (100) et, le cas échéant, la pluralité de générateurs lasers à semi-conducteurs (31 à 36) comprennent des moyens de guidage (4 à 8) selon la direction de déplacement rectiligne et des moyens (2, 3) d'entraînement de l'ensemble (1) pas à pas.

13 - Appareil selon la revendication 11, caractérisé en ce que la distance ($e_2$), entre deux faisceaux parallèles de lumière visible, et la distance ($e_1$), entre deux faisceaux de lumière visible et la lumière infra-rouge situés en regard l'un de l'autre, sont de l'ordre de 2 cm.

*Fig_1a*

10    100    20    25
101 à 106

*Fig_1b*

10    100    20    25    106    105    104    103    102    101    24    21    22    23

*Fig_1c*

20    10    106    104    102    105    103    101

0184530

30

10

40

100 ← 31 à 36

20 50

101 à 106 201 à 206

Fig.2a

20 24 36
35
34
10 33
100 32
31

21 22 23 25

Fig.2b

401 402 403 404 405 406

A

$e_1$

$e_2$

301 302 303 304 305 306

60

Fig.3

0184530

Fig-4

Fig-5

Fig-6

## Office européen des brevets

# RAPPORT DE RECHERCHE EUROPEENNE

EP 85 42 0213

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int CI 4) |
|---|---|---|---|
| A | US-A-4 174 150 (CONGLETON)<br>* Figure 1 *<br><br>- - - - - | 1-6 | A 61 N 5/06 |
|  |  |  | **DOMAINES TECHNIQUES RECHERCHES (Int. Cl.4)**<br><br>A 61 N<br>H 01 S<br>G 02 B<br>A 61 B |

Le présent rapport de recherche a été établi pour toutes les revendications

| Lieu de la recherche | Date d'achèvement de la recherche | Examinateur |
|---|---|---|
| LA HAYE | 24-02-1986 | ONILLON C.G.A. |